# EUROPEAN PATENT APPLICATION

(11) **EP 4 741 501 A1**
(43) Date of publication of application: **13.05.2026**
(21) Application number: 24212204.2
(22) Date of filing: 11.11.2024
(51) Int. Cl.: C12N 15/113, A61K 31/7088, A61K 47/54

(54) **DOSING OF OLIGONUCLEOTIDE DRUGS TARGETING MIR-21 FOR INHALATION**

(71) Applicant: RNATICS GmbH, 82152 Planegg (DE); Technische Universität München, in Vertretung des Freistaates Bayern, 80333 München (DE)
(72) Inventor: Rabe, Klaus F., 22085 Hamburg (DE); Groth, Espen E., 22335 Hamburg (DE); Frischmuth, Thomas, 22607 Hamburg (DE); Engelhardt, Stefan, 80802 München (DE); Schmidt, Johannes, 82152 Martinsried (DE)
(74) Representative: Weickmann & Weickmann PartmbB

(57) **Abstract**

The present invention relates to a formulation comprising an oligonucleotide drug which is an antagonist of microRNA-21 as an active agent for administration to a human subject in need thereof by inhalation.

## Description

### Field of the Invention

The present invention relates to a formulation comprising an oligonucleotide drug which is an antagonist of microRNA-21 as an active agent for administration to a human subject in need thereof by inhalation.

### Background of the Invention

Oligonucleotide drugs that inhibit the activity of microRNAs, e.g. microRNA-21 (miR-21), are presently under clinical development.

WO 2009/106477, the content of which is herein incorporated by reference, discloses systemic inhibitors of miR-21, called antimiR-21 for the prevention of tissue fibrosis. Based on these findings, a clinical program to develop antimiR-21 against kidney fibrosis was initiated by Regulus Therapeutics (Carlsbad, California) together with Sanofi/Genzyme. Till date, a phase 1 trial has been successfully concluded and a press release had been issued.

In 2016, a phase 2 clinical trial in Alport syndrome was approved by the FDA (HERA trial). As listed on ClinicalTrials.gov 14, the trial was a phase 2, randomized, double-blind, placebo-controlled trial to evaluate the safety, efficacy, pharmacodynamics, and pharmacokinetics of Lademirsen (antimiR-21, SAR339375). AntimiR-21 was administered systemically (s.c., 1,5 mg per injection) every week for a total of 48 weeks. According to ClinicalTrials.gov the results of the futility analysis led to the trial termination in 2022.

WO 2021/205032, the content of which is herein incorporated by reference, discloses inhibitors of miR-21 which may be administered by inhalation. These inhibitors may comprise an oligonucleotide drug, e.g., an antisense molecule directed against miR-21 conjugated to a targeting moiety for delivery to lung macrophages. No specific formulations for inhalation are described.

The publication by Beck et al. (Nature Communications (2023) 14: 4564; doi.org/10.1038/s41467-023-40185-1), the content of which is herein incorporated by reference, discloses a trimannose-conjugated antisense inhibitor of microRNA-21, designated RCS-21, as a therapeutic agent against hyperinflammation and fibrosis. No specific formulations for inhalation are described.

It was an objective of the present invention to provide a safe and effective dosing regimen for administering an oligonucleotide drug to a human subject by inhalation.

### Summary of the Invention

A first aspect of the present invention is a formulation comprising an oligonucleotide drug as an active agent for administration to a human subject in need thereof by inhalation wherein the oligonucleotide drug is an antagonist of miR-21.

In certain embodiments, the oligonucleotide drug is a conjugate comprising an oligonucleotide moiety and a targeting moiety for delivering the oligonucleotide moiety to a predetermined target cell, e.g., a macrophage. In certain embodiments, the targeting moiety is a carbohydrate moiety, e.g., a trimannose moiety.

In even more particular embodiments, the oligonucleotide drug is compound RCS-21 or a physiologically acceptable salt thereof: 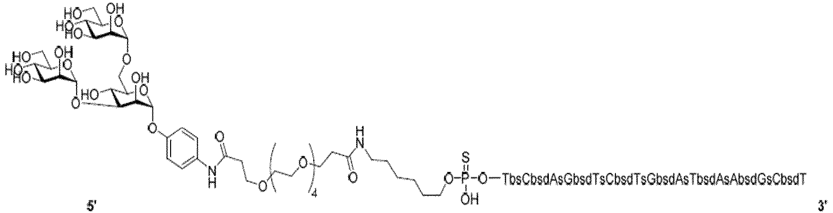
wherein the capital letters *T*, *C*, *A* and *G* in the oligonucleotide moiety represent nucleotide building blocks comprising the nucleobases thymine, 5-methyl cytosine, adenine, and guanine, respectively,
the lower-case letter s represents a phosphorothioate internucleosidic bond between two nucleotide building blocks,
the lower-case letter *b* after a capital letter denotes a locked nucleotide building block having a 2'-O-CH2-4' bridge, and
the lower-case letter *d* before a capital letter denotes a 2'-deoxyribonucleotide building block.

A further aspect of the present invention relates to a method of treating a human subject in need thereof, comprising administering to said subject a therapeutically effective amount of a formulation by inhalation, wherein the formulation comprises an oligonucleotide drug as an active agent wherein the oligonucleotide drug is an antagonist of miR-21.

### Detailed description

The present inventors have developed a dosing regimen for administering an oligonucleotide drug to a human subject by inhalation wherein the oligonucleotide drug is an antagonist of miR-21. At present, a phase 1 clinical trial based on the dosing regimen of the present invention is planned.

The term "antagonist of miR-21" as used herein relates to an oligonucleotide drug which is capable of reducing and/or blocking the physiological activity of miR-21, e.g., by acting as an antisense molecule and/or by RNA interference.

In certain embodiments, the oligonucleotide drug comprises an oligonucleotide having the nucleotide sequence (SEQ ID NO.1):

5'-TCAGTCTGATAAGCT-3'

wherein each indicated nucleotide building block is a deoxyribonucleotide building block or a modified nucleotide building block.

In certain embodiments, the modified nucleotide building block may be a 2'-modified ribonucleotide wherein the 2'-OH substituent of ribose moiety is replaced by halo, e.g., F, -C₁-C₅ alkyl, -O-C₁-C₅ alkyl, e.g. -OCH₃, -S-C₁-C₅ alkyl, -C₂-C₅ alkenyl, -O-C₂-C₅ alkenyl, -S-C₂-C₅ alkenyl, -C₂-C₅ alkynyl, -O-C₂-C₅ alkynyl, -S-C₂-C₅ alkynyl, amino including mono- or disubstituted amino, e.g., -C₁-C₅ alkyl, -C₂-C₅ alkenyl or -C₂-C₅ alkynyl substituted amino wherein each alkyl, alkenyl or alkynyl group is optionally substituted with OH, halo, cycloalkyl, cycloalkenyl, (hetero)aryl, e.g. phenyl, O-alkyl, S-alkyl, and/or amino.

In certain embodiments, the modified nucleotide building block is a bridged, e.g. a 2'-4'-bridged nucleotide building block, wherein the bridge typically has a length of 2-5 atoms, particularly 2-3 atoms including C-atoms and optionally heteroatoms such as O, N or S. In a particular embodiment, the modified building block is a locked nucleotide building block having a 2'-O-CH₂-4' bridge.

In certain embodiments, the modified nucleotide building block is a building block wherein the ring of the ribose moiety is modified, e.g. a morpholino building block, a thio-ribose building block, a 6-membered pyranose building block or a peptidic nucleic acid (PNA) building block.

In certain embodiments, the modified nucleotide building block comprises a modified internucleosidic linkage wherein the phosphoester group connecting adjacent building blocks is replaced by modified internucleosidic linkage, e.g. a phosphorothioate linkage, an alkyl phosphonate, e.g. methyl phosphonate linkage, and a borano phosphate linkage.

In particular embodiments, the oligonucleotide drug comprises an oligonucleotide having the nucleotide sequence (SEQ ID NO. 2):

5'-TCaGtCtGaTaAgCt -3'

wherein a capital letter represents a locked nucleic building block, a lower-case letter represents a deoxyribonucleotide building block, every capital C denotes 5-methyl cytosine, and all internucleosidic linkages are phosphorothioate linkages.

In certain embodiments, the oligonucleotide is conjugated to a heterologous moiety, e.g. a targeting moiety for directing the oligonucleotide drug to a target cell, particularly for selectively directing the oligonucleotide drug to a macrophage. In certain embodiments, the heterologous moiety is a peptide, lipid or carbohydrate moiety. In particular embodiments, the heterologous moiety is a carbohydrate moiety, more particularly a trimannose moiety.

In even more particular embodiments, the oligonucleotide drug is a conjugate comprising an oligonucleotide moiety of SEQ ID NO: 1 or SEQ ID NO: 2 and a carbohydrate moiety, e.g., a trimannose moiety.

The oligonucleotide may be conjugated to the heterologous moiety via a linker. In certain embodiments, the linker is attached to the 5'- end of the oligonucleotide. In certain embodiments, the linker is a polyethylene glycol containing linker.

In most particular embodiments, the oligonucleotide drug is RCS-21 or a physiologically acceptable salt thereof.

The oligonucleotide drug may be administered to a human subject by inhalation, e.g., by nasal inhalation, and particularly by oral inhalation.

In certain embodiments, the formulation is administered daily or even 2- or 3-times daily. In certain embodiments, the formulation is administered weekly, twice weekly or every second day. In certain embodiments, the formulation is administered bi-weekly, monthly, bi-monthly, tri-monthly or every 6 months.

In certain embodiments, the oligonucleotide drug is administered in a body weight dependent dosing regimen. For example, the oligonucleotide drug is administered in a dose of 0.001 mg/kg body weight to 10 mg/kg body weight, in a dose of 0.005 mg/kg body weight to 5 mg/kg body weight or in a dose of 0.01 mg/kg body weight to 2.5 mg/kg body weight per application.

In specific embodiments, the oligonucleotide drug is administered in a body weight dependent dose of 0.0625 mg/kg body weight per day, 0.3125 mg/kg body weight per day, 0.625 mg/kg body weight per day, 1.25 mg/kg body weight per day, or 2.5 mg/kg body weight per day.

In further embodiments, the oligonucleotide drug is administered in a fixed dose. For example, the oligonucleotide drug is administered in a fixed dose of 0.06 mg to 700 mg, in a fixed dose of 0.1 mg to 600 mg, in a fixed dose of 0.2 mg to 500 mg, in a fixed dose of 1 mg to 300 mg, or in a fixed dose of 5 mg to 250 mg per application, or in a fixed dose of 10 mg to 200 mg per application.

In specific embodiments, the oligonucleotide drug is administered in a fixed dose of 14.4 mg per day, 44 mg per day, 88 mg per day or 175 mg per day.

The formulation comprising the oligonucleotide drug is inhaled as an aqueous liquid, particularly as an aqueous solution, more particularly as an isotonic aqueous solution.

In certain embodiments, the formulation is a liquid ready-to-use formulation, which is stored in a pre-formulated state, e.g., as an aqueous liquid formulation. The liquid ready-to-use formulation may be prepared by dissolving the oligonucleotide drug in an aqueous liquid, e.g., an isotonic NaCl solution and filling it under suitable, e.g., sterile conditions into a container. The container is stored until use.

In certain embodiments, the formulation is a reconstitutable formulation, wherein the oligonucleotide drug is stored in a dry state, e.g. as a lyophilizate, in a container and is reconstituted by adding a reconstitution liquid, e.g., an isotonic NaCl solution before use.

The formulation of the present invention comprises the oligonucleotide drug in an amount which allows a therapeutically effective delivery. In certain embodiments, the oligonucleotide drug is present in an amount of about 1 mg/ml to about 100 mg/ml, about 5 mg/ml to about 50 mg/ml, particularly about 10 mg/ml to about 25 mg/ml and more particularly about 13 mg/ml to about 22 mg/ml, e.g. about 14 or about 20 mg/ml based on the volume of liquid.

The formulation of the present invention may further comprise NaCl. In certain embodiments, NaCl is present in an amount of about 7 mg/ml to about 11 mg/ml particularly about 8 mg/ml to about 10 mg/ml and more particularly about 9 mg/ml (based on the volume of liquid), e.g., in a ready-to-use formulation or in a reconstituted formulation.

In certain embodiments, the formulation of the present invention has a pH of about 5 to about 8, particularly about 5.0 to about 8.0, more particularly about 6 and even more particularly about 6.0. If necessary, the pH of the formulation may be adjusted, e.g., with HCl or NaOH or a buffer, e.g., a phosphate buffer, to a range of about 5.0 to about 8.0, particularly to about 6.0.

In certain embodiments, a formulation of the present invention e.g., a ready-to-use formulation or a reconstitutable formulation, consists of the oligonucleotide drug, NaCl, i.e., sodium (as ion), chloride (as ion) and water. A ready-to-use formulation consists of the oligonucleotide drug, NaCl, i.e., sodium (as ion), chloride (as ion) and water wherein all components may be provided in a single container. A reconstitutable formulation consists of the oligonucleotide drug, NaCl, i.e., sodium (as ion), chloride (as ion) and water wherein the oligonucleotide drug in a dry state, e.g., as a lyophilizate, may be provided in a first container and the reconstitution liquid may be provided in a second container. In certain embodiments, NaCl is provided with the reconstitution liquid.

In certain embodiments, the formulation of the present invention further comprises a buffer for pH adjustment, e.g., a phosphate buffer, e.g., sodium phosphate buffer, a potassium phosphate buffer or a phosphate-based saline buffer. In certain embodiments, the buffer is present in an amount of providing a formulation with a calculated osmolality of about 260 to about 360 mOsmol/L, particularly about 308 mOsmol/L, which is the calculated osmolality of a physiological sodium chloride solution. In certain embodiments, the formulation comprises 1 mM to about 30 mM potassium chloride and 1 mM to about 20 mM potassium phosphate monobasic. In certain embodiments 0.1 mM to about 2 mM sodium chloride and 5 mM to about 100 mM sodium phosphate dibasic is added. Particularly, the pH of the buffer is between about 7 to about 8, more particularly about 7.0 to about 8.0.

In certain embodiments, the formulation of the present invention, e.g., a ready-to-use formulation or a reconstitutable formulation, consists of the oligonucleotide drug, NaCl, i.e., sodium (as ion), chloride (as ion), Na-phosphate, i.e., sodium (as ion) and phosphate (including all forms of non-protonated, partially protonated and protonated phosphate), and water. A ready-to-use formulation of the present invention consists of the oligonucleotide drug, NaCl, i.e., sodium (as ion), chloride (as ion), the buffer components, e.g., sodium and phosphate (as ions) and water wherein all components may be provided in a single container. A reconstitutable formulation consists of the oligonucleotide drug, NaCl, i.e., sodium (as ion), chloride (as ion), the buffer components, e.g., sodium and phosphate (as ions) and water. The oligonucleotide drug in a dry state, e.g., as a lyophilizate, may be provided in a first container and the reconstitution liquid may be provided in a second container. In certain embodiments, NaCl and/or the buffer components are provided with the reconstitution liquid.

In particular embodiments, the formulation of the present invention is free from any surfactant. In further particular embodiments, the formulation of the present invention is free from any preservative.

In certain embodiments, the formulation is a single-dose formulation. In those embodiments, the formulation may be provided in a container comprising a single inhalation dose of the oligonucleotide drug. In further embodiments, the formulation is a multi-dose formulation. In those embodiments, the formulation may be provided in a container comprising multiple, e.g. 2, 3, 4, 5, 6, 7, 8, 9 or 10 or even more inhalation doses of the oligonucleotide drug.

The formulation of the present invention is intended for use in medicine, particularly in human medicine. The formulation is administered to the lung, bronchi and/or airways by inhalation. In certain embodiments, the formulation is a formulation for nasal inhalation. In further embodiments, the formulation is a formulation for oral inhalation.

For administration, the formulation is aerosolized to provide an aerosolized formulation which is a wet aerosol. The aerosol is administered to a subject, particularly a human subject, in a therapeutically effective amount by inhalation, e.g., by nasal inhalation or by oral inhalation. Typically, the aerosol has a droplet size of about 1-10 µm, in particular of about 3-5 µm. The aerosolized formulation may be administered to the lungs, bronchi and/or airways.

Aerosolization may comprise pressurization and/or nebulization, using a pressurized pack or a nebulizer, e.g., a mesh nebulizer or a jet nebulizer. In certain embodiments, a propellant may be added to the formulation, e.g., dichlorodifluoromethane, trichlorofluoromethane, dichlorotetrafluoroethane, carbon dioxide or other suitable gases. In certain embodiments, capsules and cartridges for use in an inhaler or insufflator may be formulated. Formulations and methods for modulating the size of droplets using nebulizer devices to target specific portions of the respiratory tract and lungs are well known to those skilled in the art.

In particular embodiments, the formulation is administered to a human subject in a therapeutically effective amount by oral inhalation.

The formulation is particularly suitable for the treatment of a disorder of the respiratory tract, particularly for the treatment of a disorder of the lower respiratory tract, more particularly for the treatment of a pulmonary disorder.

In particular embodiments, the formulation is suitable for the treatment of a disorder which is associated with, caused by and/or accompanied with pathological fibroblast and/or macrophage activity in the respiratory tract, e.g., in the lungs.

Specifically, the formulation of the present invention is suitable for the treatment of a disorder selected from pulmonary fibrosis, pneumonia, ARDS, COPD, a viral or bacterial pulmonary infection, e.g. an infection by a coronavirus such as SARS-CoV-2, or influenza virus, or Respiratory syncytial virus (RSV), or a parainfluenza virus, or a rhinovirus, or an adenovirus, or a metapneumovirus, a cancer of the respiratory tract, e.g., a lung cancer, and an inflammatory lung disease. In certain embodiments, the disorder is COVID-19 or pneumonia after an influenza infection.

The formulation may be administered as a monotherapy or, simultaneously or sequentially, as a combination therapy with a further medicament. For example, the composition provided herein will be administered in the treatment in combination with a pharmaceutical composition for the treatment of viral and/or bacterial infection(s) and anti-inflammatory treatments. Such a treatment may include but is not limited to an antiviral agent, e.g., Remdesivir, a beta-adrenergic receptor agonist, e.g., Salbutamol and/or a glucocorticoid, e.g., Dexamethasone.

Further, the present invention shall be explained in more detail by the following Examples.

### Examples

### Example 1 - Clinical trial protocol

### 1. Trial rationale

New options are urgently needed to reduce both the need for artificial ventilation and mortality in COVID 19 or other pulmonary diseases after viral infections, and to promote patient recovery.

This trial is a phase 1, randomized, double-blind, placebo-controlled trial to evaluate the safety, tolerability and pharmacokinetics of inhaled RCS-21. The trial consists of 2 parts (A and B): in Part A, a single ascending dose (SAD) design and in Part B, a multiple ascending dose (MAD) design will be employed to evaluate the above in healthy volunteers (first-in-human dose escalation).

### 1.1 Part A: Single ascending dose

A total of four ascending dose groups (DGs) are planned, with a total of n=32 participants. Each DG will consist of n=8 participants (n=6 receiving RCS-21, n=2 receiving placebo).

The respective dose levels are as follows:
- DG 1: 14.4 mg per day
- DG 2: 44 mg per day
- DG 3: 88 mg per day
- DG 4: 175 mg per day

The in-patient observation period will be 5 days (inclusive Day -1); follow-up visit will be at day 12±2; end of trial visit will be at day 30±4.

### 1.2 Part B: Multiple ascending dose

A total of two ascending dose groups (DGs) are planned, with a total of n=16 participants. Each DG will consist of n=8 participants (n=6 receiving RCS-21, n=2 receiving placebo).

The respective dose levels are as follows:
- DG 1: 44 mg per day
- DG 2: 88/175 mg per day

The decision on the high dose will be based on the data of the preceding Part A of the trial.

The treatment period will be 9 days (inclusive Day -1); follow-up visit will be at day 21±2; end-of-trial visit will be at day 37±4 days.

### 2. Objectives and Endpoints

| OBJECTIVES | ENDPOINTS | |
|---|---|---|
| Primary | | |
| To assess safety and tolerability of RCS-21 in healthy volunteers after single and multiple doses | Frequency and severity of AEs, of serious and drug-related AEs | |

| Secondary | | |
|---|---|---|
| To assess the pharmacokinetics of RCS-21 in healthy volunteers after single and multiple doses | Part A (SAD): | |
| | Summary pharmacokinetics derived by conventional non-compartmental analysis (NCA) applicable to single dose profiling: | |
| | | • Cₘₐₓ (maximum observed plasma concentration) |
| | | • Tₘₐₓ (time of Cₘₐₓ after dosing) |
| | | • AUC_{0-t last} (area under the time course of the plasma concentrations up to the last quantifiable plasma concentration) |
| | | • AUC₀₋₂₄ (area under the time course of the plasma concentrations up to 24 h after dosing, i.e. over the course of the intended dosage interval for future repeated dosing) |
| | | • AUC_{0-inf} (total area under the time course of the analyte in plasma concentrations extrapolated to infinity) |
| | | • AUC_{extrapolated} (relative extent of extrapolation) |
| | | • T_{½} (apparent terminal disposition half-life) and I_{z} (apparent terminal elimination rate constant) |
| | Part B (MAD): | |
| | Summary pharmacokinetics derived by conventional non-compartmental analysis (NCA). | |
| | After the first dose of multiple ascending dosing: | |
| | | • C_{max,1} (maximum observed plasma concentration) |
| | | • T_{max,1} (time of Cₘₐₓ after dosing) |
| | | • AUC_{0-tz,1} (area under the time course of the plasma concentrations up to the last quantifiable plasma concentration) |
| | | • AUC_{0-24,1} (area under the time course of the plasma concentrations up to 24 h after first dosing, i.e. over the course of the dosage interval for further repeated dosing) |
| | Shortly before the last dose of multiple ascending dosing: | |
| | | • C_{trough,ss} (trough concentration shortly before the last of the repeated doses) |
| | After the last dose of multiple ascending dosing: | |
| | Summary pharmacokinetics derived by conventional non-compartmental analysis (NCA) applicable to repeated dose profiling (dosage interval t) in presumed steady-state (ss): | |
| | | • C_{max,ss} (maximum observed plasma concentration) |
| | | • T_{max,ss} (time of Cₘₐₓ after dosing) |
| | | • C_{min,ss} (minimum observed plasma concentration) |
| | | • C_{avg,ss} (average plasma concentration) |
| | | • AUC_{0-24,ss} (area under the time course of the plasma concentrations up to 24 h after last dosing, i.e. over the course of the dosage interval) |
| | | • PTF (Peak-to-Trough Fluctuation) |
| | | • By combining data from the last dose with the data from the first dose: LF (linearity factor); ACR_{Cmax} (accumulation ration for Cₘₐₓ); ACR_{AUC} (accumulation ratio for AUC₀₋ₒ₋ₜ) |

### 3. Trial population

The trial will be performed in healthy volunteers.

### 3.1 Inclusion criteria

1. Able and willing to give written informed consent.
2. Male or female aged 18 to 64 years (inclusive).
3. Women will be considered for inclusion if they are:
   a. Not pregnant, as confirmed by pregnancy test and not breastfeeding.
      AND
   b. WOCBP must use a highly effective method of birth control that result in a low failure rate of less than 1% per year when used consistently and correctly - according to recommendations by the European Heads of Medicines Agencies - from at least 14 days before the first administration of study medication until 30 days after the last administration of study medication.
      OR
   c. Of non-childbearing potential defined according to the Clinical Trial Facilitation Group (CTFG) document "Recommendations related to contraception and pregnancy testing in clinical trials"
4. Male participants with female partner(s) of childbearing potential are eligible to participate in the study if they agree to the following during treatment and until 30 days after the last administration of study medication:
   a. Inform any and all partner(s) of their participation in a clinical drug study and the need to comply with contraception instructions as directed by the investigator.
   b. Male participants are required to use a condom during treatment and until 30 days after the last administration of study medication.
   c. Female partners of male participants should consider use of effective methods of contraception during treatment and until 30 days after the last administration of study medication.
   d. Sperm donation is not allowed during treatment and until 30 days after the last administration of study medication.
5. Healthy participants according to the assessment of the investigator, as based on a complete medical history including a physical examination, vital signs, 12-lead Electrocardiogram (ECG), pulmonary function testing and clinical laboratory tests.
6. Body Mass Index (BMI) of 18.5 to 31.9 kg/m² (inclusive).
7. Ability to inhale in an appropriate manner. (e.g. as confirmed in the inhalation training using the nebulizer device with a placebo medication at the screening visit).
8. Non-smokers (including e-cigarette) or ex-smokers (with less than 10 pack years and stopped smoking for at least 5 years prior to screening visit).
9. Normal pulmonary function with Forced Expiratory Volume in the first second (FEV1) ≤ 80 % of predicted normal at screening visit. Calculations will be based on the Global Lung Function Initiative (GLI 2012) formula.

### 4. Duration of individual participation

In Part A, the total planned duration of individual participation is 41 to 58 days. In Part B, the total planned duration of individual participation is 48 to 69 days. An individual trial participation is completed when the participant has completed the end of trial visit.

### 5. Randomization

Randomization will be performed within each trial part (A and B) separately for each dose level. At Visit 3, participants will be randomly assigned to receive either RCS-21 or placebo according to a randomization scheme developed for the trial.

### 6. Visit schedule

### 6.1 Trial Part A

The screening period will consist of 2 visits.

At V1, participants will be subjected to the following medical procedures including medical history and demographics, complete physical examination, alcohol, smoking and drug tests, pregnancy test in females, body size (i.e., height and weight) and vital signs, 12-lead ECG, blood and urine sampling for safety laboratory, spirometry, QuantiFERON test, checking In/exclusion criteria.

At V2, participants will be subjected to the following medical procedures including alcohol test, spirometry, vital signs, brief physical examination, and pregnancy test in females. Prior to discharge, occurrence of any Adverse Effects (AEs) or use of Concomitant Medication (ConMed) during the trial visit will be recorded.

The treatment period will consist of a single Visit 3 scheduled as an inpatient treatment from Day -1 to Day 4.

During V3 the following procedures will be performed: brief physical examination, vital signs, 12-lead ECG, spirometry, checking In/exclusion criteria, and checking AE/ConMed. Participants will be randomized, an initial PK blood sample, and administration of IMP/placebo will be performed. After administration of IMP/placebo the following procedures will be performed: PK blood sampling at pre-defined times, spirometry, vital signs and 12-lead ECG and occurrence of any AEs or use of ConMed will be recorded.

The Follow Up period consists of Visit 4 and Visit 5. The following procedures will be performed: brief physical examination, spirometry, vital signs, 12-lead ECG, safety laboratory, and pregnancy test in females. Prior to discharge, occurrence of any AEs or use of ConMed will be recorded.

### 6.2 Trial Part B

The screening will consist of 2 visits which will be conducted substantially as described in section 6.1 supra.

The treatment period comprises Visit 3 scheduled as an inpatient treatment from Day - 1 to Day 8 substantially as described in section 6.1 supra. Administration of IMP/placebo will happen daily for 7 consecutive days.

A PK blood draw will be performed after each dosing. Further parameters will be determined substantially as described in section 6.1 supra.

The Follow Up period consists of Visit 4 and Visit 5. The procedures will be conducted substantially as described in section 6.1 supra.

## Claims

1. A formulation comprising an oligonucleotide drug as an active agent for administration to a human subject in need thereof by inhalation wherein the oligonucleotide drug is an antagonist of miR-21.

2. The formulation of claim 1 for the use of claim 1, which is a liquid formulation.

3. The formulation of claim 1 or 2 for the use of claim 1, wherein the formulation is an aqueous formulation, particularly an aqueous isotonic solution.

4. The formulation of any one of claims 1-3 for the use of claim 1 wherein the oligonucleotide drug comprises an oligonucleotide having the nucleotide sequence (SEQ ID NO.1):
5'-TCAGTCTGATAAGCT-3'
wherein each indicated nucleotide building block is a deoxyribonucleotide building block or a modified nucleotide building block.

5. The formulation of any one of claims 1-4 for the use of claim 1, wherein the oligonucleotide drug comprises an oligonucleotide having the nucleotide sequence (SEQ ID NO. 2):
5'-TCaGtCtGaTaAgCt -3'
wherein a capital letter represents a locked nucleic building block, a lower case letter represents a deoxyribonucleotide building block, every capital C denotes 5-methyl cytosine, and all internucleosidic linkages are phosphorothioate linkages.

6. The formulation of any one of claims 1-5 for the use of claim 1, wherein the oligonucleotide drug is conjugated to a macrophage-targeting moiety, particularly to a trimannose moiety.

7. The formulation of any one of claims 1-6 for the use of claim 1, wherein the oligonucleotide drug is RCS-21 or a physiologically acceptable salt thereof: 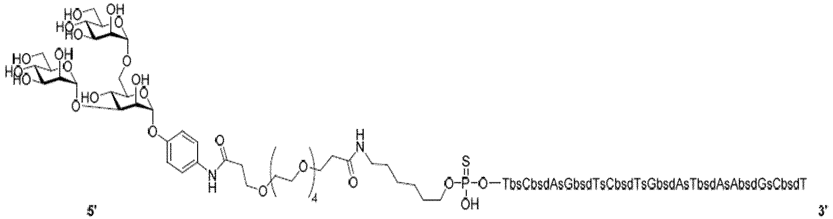
wherein the capital letters T, C, A and G in the oligonucleotide moiety represent nucleotide building blocks comprising the nucleobases thymine, 5-methyl cytosine, adenine, and guanine, respectively,
the lower-case letter s represents a phosphorothioate internucleosidic bond between two nucleotide building blocks,
the lower-case letter b after a capital letter denotes a locked nucleotide building block having a 2'-O-CH₂-4' bridge, and
the lower-case letter d before a capital letter denotes a 2'-deoxyribonucleotide building block.

8. The formulation of any one of claims 1-7 for the use of claim 1, wherein the formulation is administered by oral inhalation.

9. The formulation of any one of claims 1-7 for the use of claim 1 or 8, wherein the formulation is administered once daily, each second day, twice weekly or once weekly.

10. The formulation of any one of claims 1-7 for the use of claim 1, 8 or 9, wherein the oligonucleotide drug is administered in a body weight dependent dosing regimen. For example, the oligonucleotide is administered in a dose of 0.001 mg/kg body weight to 10 mg/kg body weight, in a dose of 0.005 mg/kg body weight to 5 mg/kg body weight or in a dose of 0.01 mg/kg body weight to 2.5 mg/kg body weight per application.

11. The formulation of any one of claims 1-7 for the use of claim 1, 8 or 9, wherein the oligonucleotide drug is administered in a fixed dose of 0.06 mg to 700 mg, 0.2 mg to 200 mg, in a fixed dose of 0.5 mg to 150 mg, or in a fixed dose of 1 mg to 50 mg per application.

12. The formulation of any one of claims 1-7 for the of any one of claims 1 or 8-10, wherein the formulation is administered for a time period of at least one week and up to 1 year or longer.

13. The formulation of any one of claims 1-7 for the of any one of claims 1 or 8-12, wherein the human subject suffers from a disorder selected from pulmonary fibrosis, pneumonia, ARDS, COPD, a viral or bacterial pulmonary infection, e.g. an infection by a coronavirus such as SARS-CoV-2 or influenza virus, a cancer of the respiratory tract, e.g., a lung cancer, and an inflammatory lung disease.

14. The formulation of any one of claims 1-7 for the use of any one of claims 1 or 8-13, for use in the selective delivery of the active agent to lung macrophages.

15. A method of treating a human subject in need thereof, comprising administering to said subject a therapeutically effective amount of a formulation by inhalation, wherein the formulation comprises an oligonucleotide drug as an active agent wherein the oligonucleotide drug is an antagonist of miR-21.
